# EUROPEAN PATENT SPECIFICATION

(11) **EP 1 695 684 B1**
(45) Date of publication and mention of the grant of the patent: **15.02.2012**
(21) Application number: 05101465.2
(22) Date of filing: 25.02.2005
(51) Int. Cl.: A61J 15/00, A61M 25/10, A61B 5/03

(54) **Enteral feeding catheter, computer system and computer program for operating the feeding catheter**
Katheter für die enterale Ernährung, Computerprogramm und Computersystem zum Betreiben des Katheters
Cathéter pour l'alimentation entérale, système informatique et programme informatique pour faire fonctionner le cathéter

(43) Date of publication of application: 30.08.2006
(73) Proprietor: Pulsion Medical Systems AG, 81829 München (DE)
(72) Inventor: Pfeiffer, Ulrich J., Dr., 81667, München (DE); Knoll, Reinhold, 81543, München (DE); Malbrain, Manu, Dr., 3360, Lovenjoel (BE)
(74) Representative: Kehl, Günther

(56) References cited:
- WO-A-02/068036
- US-A- 3 055 371
- US-A- 3 483 859
- US-A- 4 381 011
- US-A- 5 152 776
- US-A- 5 314 409
- US-A- 5 433 216
- US-A- 5 462 528
- US-A- 5 718 685
- US-A1- 2001 053 920

## Description

The invention relates to an enteral feeding catheter for channelling flowable nutrient into a patient's digestive tract, such as into patient's stomach, duodenum or jejunum, said catheter being adapted to be advanced through patient's esophagus, said catheter having a proximal end, a distal end, and a first lumen, said first lumen having an opening at said proximal end adapted to be connected to a supply for receiving said nutrient from said supply and channelling said nutrient from said proximal end to said distal end, said distal end being provided with several radial outlets for the exit of said nutrient.

Feeding catheters of this type are used for instance in feeding of a patient who is unable to swallow nutrients in the natural manner or in postoperative or critical care. Especially in critical care it is important to monitor the intra-abdominal pressure in order to avoid intra-abdominal hypertension or abdominal compartment syndrom, which may impede blood circulation and perfusion in the abdominal region and may be hazardous to the patient.

DE 35 00 822 A1 discloses a device for measuring the pressure in the human or animal body. The device comprises a balloon which is attached at the distal end of a multi-luminal catheter and which is only partially filled with a gaseous medium. The pressure in the balloon is transferred over a lumen of the catheter to a pressure gauge. DE 35 00 822 A1 does not disclose an enteral feeding catheter.

US 5433216 discloses a nasogastric tube including a balloon for intra-abdominal pressure measuring.

It is disclosed an enteral feeding catheter device which allows reliable continuous determination of patient's intra-abdominal pressure and keeps the additional burden related to the pressure measurement for the critical ill patient as low as possible.

It is an object of the invention to provide a computer system and a computer program which allow for calibration and recalibration of the measurement equipment and allow continuous determination of the intra-abdominal pressure with good accuracy.

An enteral feeding catheter as mentioned above is provided with a second lumen, adapted to be connected to a source/sink of gas and a gas pressure gauge at its proximal end and being connected to an inflatable and deflatable distal balloon provided on said catheter body near said distal end. The balloon will be named "distal balloon" in the following just for the sake to give it a name and to distinguish it from other balloons (described later) which are situated proximally with respect to the distal balloon. "Distal" must not be understood in the sense that the distal balloon is situated at the very distal end of the catheter body.

The distal balloon has preferably a high degree of gastightness allowing relatively long recalibration intervals.

Preferably the enteral feeding catheter has a third lumen adapted to be connected to a source/sink of air and a gas pressure gauge at its proximal end and being connected to an inflatable and deflatable proximal balloon provided on said catheter body at an axial position such that said second balloon is positioned in the patient's thorax when the catheter is inserted. The balloon will be named "proximal balloon" in the following just for the sake to give it a name and to distinguish it from the distal balloon which is situated distally with respect to the proximal balloon. "Proximal" must not be understood in the sense that the balloon is situated close to the proximal end of the catheter body. Providing the catheter with a proximal balloon which is situated in the thorax region of the patient, i.e. above the diaphragma when the catheter is properly inserted in place, allows to monitor not only the intra-abdominal pressure but also the intra-thoracic pressure which has an importance in monitoring cardiovascular parameters of the patient which cannot be overestimated.

According to another example the enteral feeding catheter has a fourth lumen adapted to be connected to a source/sink of gas or fluid at its proximal end and being connected to a blocking balloon provided on said catheter body at an axial position such that said blocking balloon is positioned in patient's stomach when the catheter is inserted. This blocking balloon, which is preferably filled with air or water, is very useful to assist in placing the catheter precisely and correctly without applying x-ray imaging. The catheter is advanced through the patient's esophagus with the blocking balloon being empty until the operator can be sure that the blocking balloon has entered the stomach. The balloon is then filled with air or water and the catheter is withdrawn until the blocking balloon abuts the cardia of patient's stomach, thus ensuring that the catheter is placed precisely with respect to the stomach. Additionally the blocking balloon prevents the catheter from being withdrawn further from its place. In order to avoid accidental overpressure the pressure inside the balloon could be limited and/or monitored. An overpressure would indicate falsely positioning of the blocking balloon in the esophagus.

Alternatively or additionally the feeding catheter may be provided with several radioopaque marking rings distributed over its length and/or nearby the balloons and/or a radioopaque stripe extending axially over its length to allow observing the placement of the catheter under X-ray-imaging.

Insertion marks distributed over the length of the catheter may inform the operator on how far the catheter has been advanced into the patient's body.

Additionally the feeding catheter may be provided with two or more electrodes placed near the distal end of the catheter for impedance measurement to determine the acidity of the gastric juice and to carry out gastric volume measurements. The gastric volume is negative correlated with the bio-electrical impedance i.e. impedance decreases if gastric volume increases. The gastric volume could be calculated with a empirical estimated function of the impedance. Additionally or alternatively the feeding catheter may provide two or more electrodes sufficiently proximal before or after the proximal balloon 8. These electrodes should be placed above diaphragm in the esophagus in order to estimate transthoracic bioimpedance which measures cardiac stroke volume and cardiac output and thoracic fluid content. Additionally the feeding catheter may provide pulse densitometry means placed in the esophagus in order to measure arterial oxygen saturation or indo cyanine green dye concentration. It was found that this would be more reliable than common non invasive pulse densitometry means e.g. on the finger or ear.

A computer, as claimed in claim 1, which provides for calibration and recalibration of the measurement equipment and allows continuous determination of the intra-abdominal pressure with good accuracy comprises first connection means adapted to connect said computer system to pump driving means adapted to drive a pump for increasing/decreasing the gas volume inside at least one balloon of an enteral feeding catheter and second connection means adapted to connect said computer system to a pressure gauge adapted to determine the pressure p prevailing in said at least one balloon, calculation means for calculating the mathematical derivative dp/dV of pressure p with respect to volume V displaced by the pump and accessing means to access executable instructions to cause said computer system to cause said pump driving means to adjust the gas volume inside said balloon for initial setting such that said mathematical derivative dp/dV of pressure p with respect to volume V is zero or as close to zero as possible.

Preferably the computer further comprises third connection means adapted to connect said computer system to valve means adapted to connect for initial settings said pressure gauge to ambient air and disconnect it from ambient air, said accessing means access instructions to cause said computer system to connect said pressure gauge to ambient air and to adjust said pressure gauge to zero.

Advantageously the computer further comprises alarm means alerting of a blocked or occluded catheter lumen, in case that the absolute mathematical derivative dp/dV of pressure p with respect to volume exceeds a certain upper threshold at a certain pressure.

The computer may further comprise alarm means alerting of a leakage in case that the absolute mathematical derivative dp/dV of pressure p with respect to volume V fails to reach a lower threshold at a certain pressure.

A computer program, as claimed in claim 5, which provides for calibration and recalibration of the measurement equipment and allows continuous determination of the intra-abdominal pressure with good accuracy comprises instructions executable by a computer system to cause said computer system for initial settings to drive a pump for increasing/decreasing the gas volume inside at least one balloon of said enteral feeding catheter and collecting pressure readings of pressure p prevailing in said at least one balloon, calculating the mathematical derivative dp/dV of pressure p with respect to volume V and adjusting the gas volume inside said balloon such that said mathematical derivative dp/dV of pressure p with respect to volume V is zero or as close to zero as possible.

Preferably the computer program comprises the steps of connecting said pressure gauge to ambient air, adjusting said pressure gauge to zero and disconnecting said pressure gauge from ambient air.

Advantageously the computer program comprises the step of activating alarm means alerting of a blocked or occluded catheter lumen, in case that the absolute mathematical derivative dp/dV of pressure p with respect to volume V exceeds a certain upper threshold at a certain pressure.

Preferably the computer program further comprises the step of activating alarm means alerting of a leakage in a catheter lumen, in case that the absolute mathematical derivative dp/dV of pressure p with respect to volume V fails to reach a lower threshold at a certain pressure.

Preferably the computer program further comprises the steps of continuously collecting pressure readings after initialization has been accomplished.

Preferably the computer program further comprises the step of activting an alarm when the pressure exceeds a certain threshold.

The invention including its construction and method of operation will be illustrated in the drawings in which
Fig. 1 is an overall view of an example of a feeding catheter;
Fig. 2 is a longitudinal sectional view of the proximal part of the catheter of Fig. 1;
Fig. 3 is a longitudinal sectional view of the middle part of the catheter of Fig. 1 taken along line III-III of Fig. 5;
Fig. 4 is a longitudinal sectional view of the distal part of the catheter of Fig. 1 taken along line IV - IV of Fig. 6;
Fig. 5 is a cross sectional view of the catheter taken along, line V -V in Fig. 3;
Fig.6 is a cross sectional view of the catheter taken along line VI -VI in Fig. 6;
Fig. 7 is a cross sectional view of the catheter taken along line VII -VII in Fig. 4;
Fig. 8 is a cross sectional view of the catheter taken along line VIII in Fig. 2;
Fig. 9 is a diagrammatic view of a computerized system for operating the catheter of the invention;
Fig. 10 is a diagram showing the pressure P measured with a pressure gauge as a function of the displacement x of a piston;
Fig. 11 is an overall view of a further example of a feeding catheter.

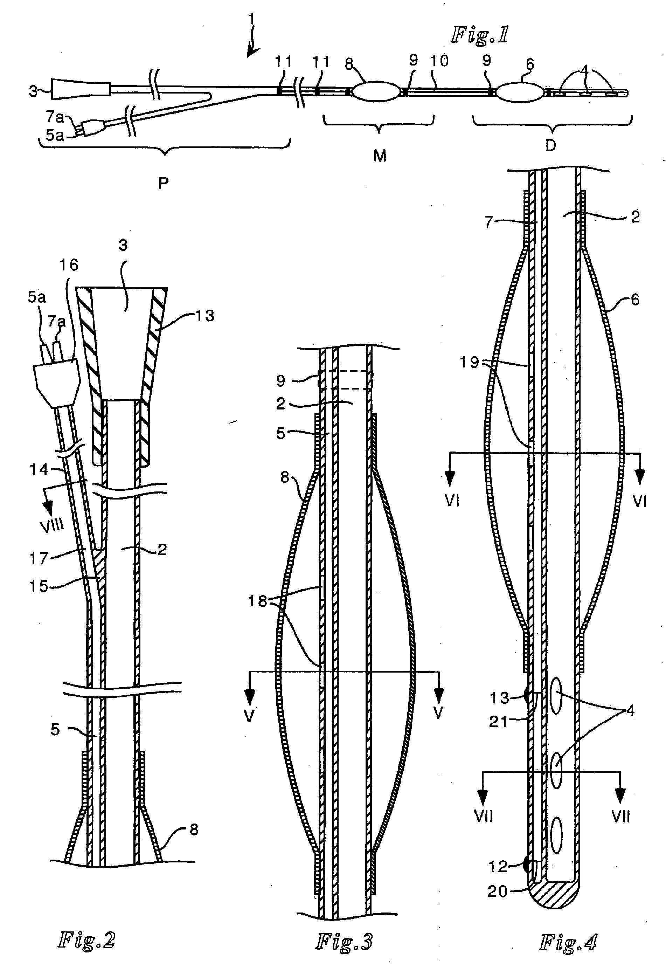

Fig. 1 shows an example of a feeding catheter. The catheter 1 has three sections, namely a proximal section P, a middle section M and a distal section D. The catheter has at its proximal end an opening 3 by means of which which it may be connected to a supply of nutrient, which may be hanged at a infusion stand (not shown). There are two further openings, namely openings 5a and 7a, to which sources/sinks of gas pressure may be connected.

In the middle part M the body of the catheter 1 is surrounded by a proximal balloon 8 which is internally connected to the opening 5a, so that the proximal balloon 8 may be inflated by pressing air into the opening 5a or may be deflated by sucking air out of opening 5a which will be explained in detail later. The balloon 8 is named "proximal balloon" just for the sake to give it a name and to distinguish it from a balloon which is situated distally with respect to the proximal balloon which will be described later. "Proximal" is not to be understood that the balloon is situated at the proximal end of the catheter body.

In the distal part D the body of the catheter 1 is surrounded by a distal balloon 6 which is internally connected to the opening 7a, so that the distal balloon 6 may be inflated by pressing air into the opening 7a or may be deflated by sucking air out of the opening 7a which will be explained in detail later. The balloon 6 is named "distal balloon" just for the sake to give it a name and to distinguish it from the proximal balloon 8 which is situated proximally with respect to the distal balloon 6 mentioned before. "Distal" must not be understood that the balloon is situated at the very distal end of the catheter body.

Distally with respect to the distal balloon 6 there are provided radial openings 4 which serve as outlets to allow flowable nutrients or medications, which have been introduced into the catheter 1 through its opening 3, to exit into patient's digestive tract.

Before and after balloons the catheter has radio opaque marking rings 9, an axially extending radio opaque stripe 10, and optically visible insertion marks 11, supporting the operator in determining the position of the catheter in patient's body, especially during the time when it is advanced through the digestive tract of the patient. The marks 10 extent from balloon 8 up to the Y-junction 15 at equal distances (e.g. 5 cm).

The details of the structure of catheter 1 will become more apparent from figure 1 which shows a longitudinal section of the proximal part of the catheter 1. The proximal opening 3 of the catheter 1 is provided with a conical connector 13 to which a supply line for nutrients may be connected. The supply line (not shown) will disconnect from the connector 13 automatically if an excessive pulling force is exerted thereby protecting the patient from being injured. Downstream or distally of the connector 13 there is a first lumen 2, which serves to guide flowable nutrients or medicine down into patient's digestive tract.

A side hose 14 is connected to the main part of the catheter 1 via a Y-junction 15. The distal end of the side hose 14 is closed by a connector 16, which has two openings 5a and 7a. The side hose 14 is connected to a second lumen 5 and a third lumen which is not visible in figure 2. Inside the side hose 14 there is provided a lumen 5b which connects the opening 5a to the second lumen 5. There is a further lumen 7b in the side hose 14, which connects the second opening 7a to the third lumen 7 which is however not visible in the sectional view of figure 2. Downstream of the Y-junction 15 the second lumen 5 is parallel to the larger first lumen 2.

As may be best understood from figure 3 the lumen 5 is connected to the proximal balloon 8. In the region where the body of the catheter 1 is surrounded by the proximal balloon 8 the outer wall of the second lumen 5 has radial openings 18 which allow for gas flow between the second lumen 5 and the proximal balloon 8. As a result the interior of the proximal balloon 8 is connected to the opening 5a (figure 2) so that the proximal balloon 8 can be inflated by blowing air or another gas into the opening 5a.

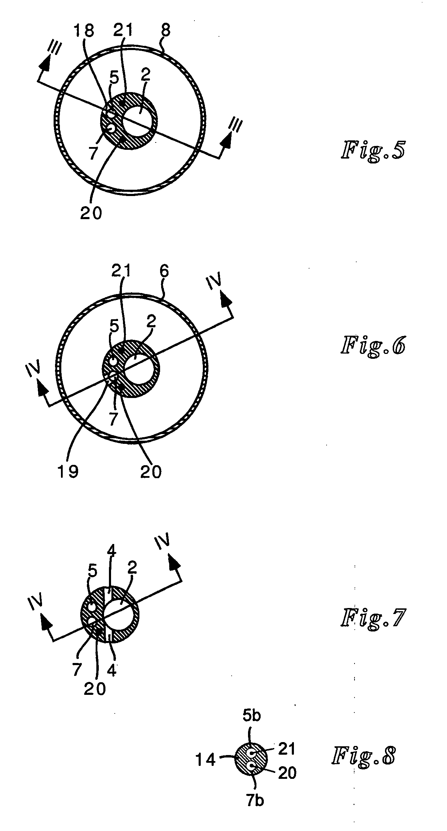

Figure 4 shows the distal part D of the catheter 1. It is to be obseved that - although the drawing of figure 4 looks similar to that of figure 3 - the plane of section is different to that of figure 3, which can be best understood from figures 5 and 6 in which the respective planes of section are indicated by arrows marked with "III" and "IV". As can be seen the plane of section for figure 3 traverses the second lumen 5 diametrally, while the plane of section of figure 4 traverses the third lumen 7 diametrally.

As becomes apparent from figure 4, the third lumen 7 is connected to the interior of the distal balloon 6 via radial openings 19. The interior of the distal balloon 6 communicates with the opening 7a and can hence be inflated and deflated through that opening 7a.The surface inside the balloons 6 and 8 is roughened or profiled in a way to ensure complete emptening of gas at deflation.

Close to the distal tip of the catheter 1 the first lumen 2 is provided with several radial outlets 4 which allow nutrient to flow out of the first lumen 2. At the outer wall of the catheter 1 there are further provided two electrodes 12 and 13 spaced apart from each other. The electrodes are connected by electric wires 20 and 21 to the connector 16 (figure 2).

Figure 5 is a cross sectional view of the catheter taken along line V -V in figure 3. Figure 5 shows the catheter with its first lumen 2, second lumen 5, third lumen 7 and embedded connection wires 20, 21 for the electrodes 12, 13 shown in figure 4. Alternatively the wires 20 and 21 could be placed inside the lumina 5 and 7. The second lumen 5 is connected via openings 18 to the interior of the proximal balloon 8, while there is no such connection between the third lumen 7 and proximal balloon 8.

Figure 6 is a cross sectional view of the catheter taken along line IV -IV in figure 4. Figure 6 shows the catheter with its first lumen 2, second lumen 5, third lumen 7 and connection wires 20, 21 for the electrodes 12, 13 shown in figure 4. The third lumen 7 is connected via openings 19 to the interior of the distal balloon 6, while there is no such connection between the second lumen 5 and the distal balloon 6.

Figure 7 is a cross sectional view of the catheter taken along line VII -VII in figure 4. Figure 7 shows the catheter with its first lumen 2, second lumen 5, third lumen 7 and connection wire 20 for the electrode 12 shown in figure 4. The first lumen 2 has openings 4 allowing nutrient to flow out into patient's digestive tract.

Figure 8 is a cross sectional view of the catheter taken along line VIII -VIII in figure 2. Figure 2 shows the side hose 14 which is connected to the main part of the catheter 1 via a Y-junction 15 (figure 2). Inside the side hose 14 there are is provided the lumen 5b which connects the opening 5a to the second lumen 5 (figure 2). There is a further lumen 7b in the side hose 14, which connects the second opening 7a (figure 2) to the third lumen 7 (figure 4).

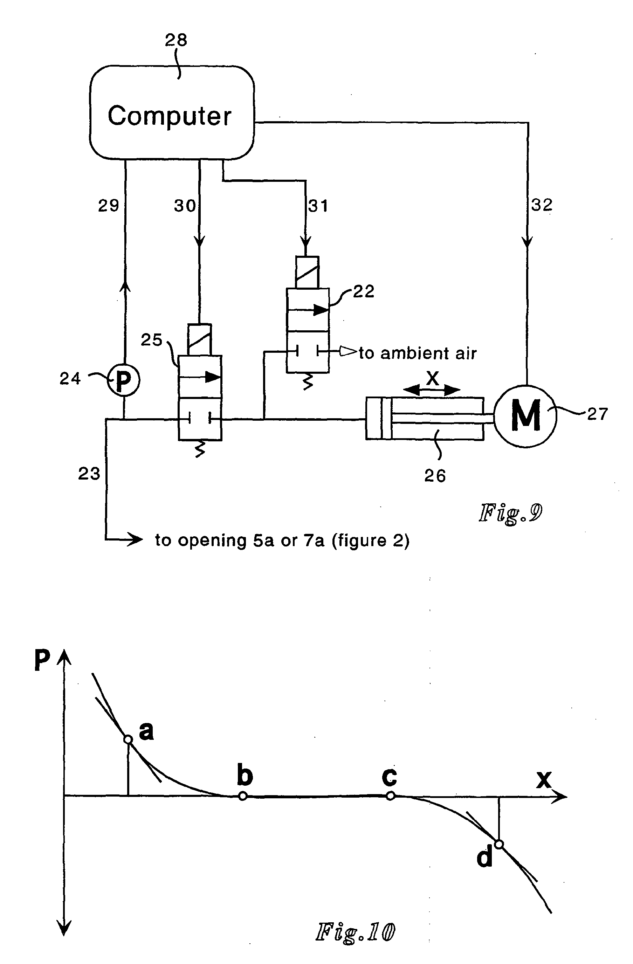

Figure 9 is a diagrammatic view of a computerized system for operating the catheter 1 of the invention. The catheter 1 is connected via its opening 5a to air conduit 23. The air conduit 23 is connected to a pressure gauge 24, by which the pressure in the air conduit 23 can be measured continuously. The air conduit 23 is further connected over a first electromagnetic valve 25 to a piston pump 26 driven by a motor 27. The air conduit 23 is further connected to a second electromagnetic valve 22, by which the conduit may be connected to ambient air. A computer 28 is electrically connected via line 29 to the pressure gauge 24, via line 30 to the first electromagnetic valve 25, via line 31 to the second electromagnetic valve 22 and via line 32 to the electric motor 27. By means of these connections the computer 28 is able to collect pressure readings from the pressure gauge 24, to open and close the first and second valves 25 and 22 and to activate the motor 27 in order to advance or retract the piston of the piston pump 26. The catheter 1 may be connected via its opening 7a to an identical system or a switch valve (not shown) may be provided to connect the system shown in figure 9 selectively to the opening 5a and 7a of the catheter 1.

The catheter system is operated as follows:
The catheter 1 is advanced with its distal tip ahead through the mouth or the nose of a patient into the esophagus of the patient until its tip reaches the stomach, the duodenum or the jejunum of the patient depending on the medical requirements. The axial position of the distal balloon is such that the distal balloon will be placed in the stomach (or duodenum or jejunum), while the axial position of the proximal balloon on the body of the catheter 1 is such that it will be placed in the patient's thorax. The movement of the catheter 1 is monitored by means of the radioopaque marking rings 9, radioopaque stripe 10 and/or by counting visible insertion marks 11 outside the patient.

Once the distal tip of the catheter 1 has reached its final destination in the patient's digestive tract, the computerized system as shown in figure 9 is connected to the opening 5a. The computer program that runs on the computer 28 causes the first and the second electromagnetic valves 22 and 25 to open, so that the air conduit 23 is connected to ambient air. Then the piston 26 is moved to a defined e.g. middle position and pressure gauge 24 is set to zero. The computer 28 then causes the electromagnetic valve 22 to close in order to disconnect the air conduit 23 from ambient air. The computer causes the motor 27 to drive the piston of piston pump 26 in forward direction so that air is blown into the the proximal balloon 8 via air conduit 23, opening 5a, lumen 5b, lumen 5 and openings 18, while taking pressure readings from the pressure gauge 24 and calculating the mathematical derivative dp/dV of pressure p with respect to volume V permanently. Instead of calculating dp/dV equally dp/dx may be calculated, where x is the position of the piston of the piston pump 26. The curves dp/dV and dp/dx have the same characteristic form and differ only by a constant scaling factor which corresponds to the (constant) cross section of the piston. The same applies for variables x and V, so that it does not make any difference if V or x is recorded.

During the inflation process the pressure inside the proximal balloon 8 increases and the balloon 8 unfolds. After the balloon 8 has been unfolded the pressure inside the balloon increases rapidly, in other words the absolute amount of mathematical derivative of pressure p with respect to displacement x of the piston is high and exceeds a predetermined threshold at a certain positive pressure, causing the program to stop the inflation process and to start a deflation, in other words the piston will be moved in the opposite direction whereby the balloon 8 is evacuated. At the beginning of the deflation process the pressure falls rapidly until the balloon 8 begins to collapse. During the process of collapsing the balloon 8 the pressure changes only slightly or remains constant, in other words dp/dV and equally dp/dx are zero or at least very close to zero. When the balloon 8 has reached the state of being completely collapsed further movement of the piston will cause rapid pressure drop, in other words dp/dV and dp/dx respectively will have large absolute amounts. When the absolute amount of the derivative with respect to volume exceeds a predetermined threshold at a certain negative pressure the evacuation action will be stopped by the computer 28.

The diagram shown in figure 10 reflects the evacuation process. At the beginning (point a) the pressure drops rapidly and remains approximately constant between points b and c. Further evacuation, i.e. further withdrawal of the piston of the piston pump 26 causes an increasingly rapid pressure drop until point d is reached.

The pressure inside the stomach can however be assessed reliably when it is independent of the filling state of balloon 8, i.e. in the range between point b and point c.

After having collected the pressure readings and having calculated the derivative dp/dx (or dp/dV) the computer determines a position between points b and d, preferably the middle between points b and d, and moves the piston to this position to finalize the initial setting of the system.

In order to minimize leakage, valve 25 is closed after this calibration phase. If leakage is negligible valve 25 could be ommitted. Then the patient's thoracic pressure can be assessed continuously. After some time due to inavoidable leakage e.g. of the balloon a recalibration may become indicated.

Assumed now that the balloon 8 or any other part as for example the connector 16 has a leakage. In this case the pressure p and its mathematical derivative dp/dV with respect to volume V will remain low during the process of inflation and the pressure and its derivative will remain below a predetermined threshold. This will be recognized by the computer system and an alarm will be given.

Further it may occur that a lumen may be occluded or intentionally blocked because the catheter used has only one balloon. This situation as well will be recognized by the computer system because in this case the pressure during the inflation process will go high very rapidly.

Needless to say that the initial adjustment for the distal balloon 6 is done in the same way by connecting the opening 7a to the computerized system and proceeding in the same way as described above with respect to the proximal balloon 8 in order to determine the patient's intra abdominal pressure. The computer may then calculate also the difference between abdominal and thoracal pressure and monitor this difference value.

While an example has been described in which a catheter with two balloons is employed it goes without saying that only one balloon may be employed if the measurement of the pressure at only one site in patient's body is regarded to be sufficient. One of the two balloons and the catheter lumen pertaining to it may be left away.

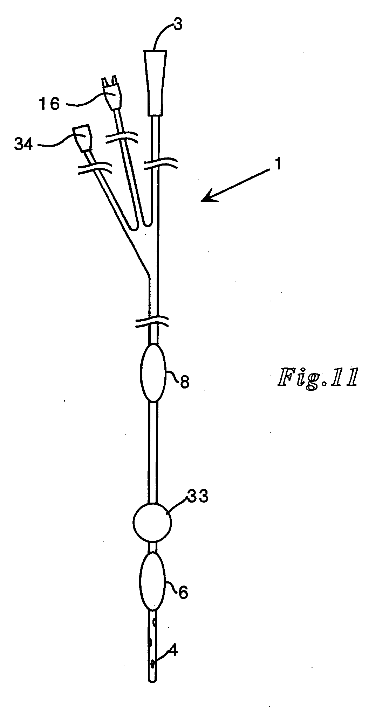

Figure 11 is an overall view of a further example of the feeding catheter. The catheter has a proximal balloon 8 and a distal balloon 6 and is constructed in the same way as the catheter described with reference to the drawings 1 to 8. However, the catheter is provided with an additional balloon 33 which serves as a blocking balloon. The balloon 33 is connected to an additional lumen (not shown in the drawing) in the same way as described with regard to balloons 6 and 8 of the first embodiment. In the embodiment of figure 11 the additional connector 34 is provided with one further opening allowing to supply a liquid under pressure into the balloon 33 and to withdraw it from the balloon 33.

The catheter of figure 11 is applied as follows:
The catheter 1 is advanced with its distal tip ahead through the mouth or the nose of a patient into the esophagus of the patient until its tip reaches the stomach, the duodenum or the jejunum of the patient depending on the medical requirements. The axial position of the distal balloon 6 is such that the distal balloon 6 as well as the blocking balloon 33 will then be situated in the stomach. The movement of the catheter 1 is monitored by means of counting the visible insertion marks 11 to make sure that the blocking balloon has entered the stomach. Then the blocking balloon 33 is filled with water and the catheter will be retracted until the blocking balloon 33 abuts the cardia of the patient's stomach, thus ensuring that the catheter is placed precisely with respect to the stomach and balloon 8 will be situated in the patient's thorax. Additionally the blocking balloon prevents the catheter 1 from being withdrawn from its place. This blocking balloon is very useful to assist in placing the catheter precisely and correctly without applying X-ray imaging.

The further operation of the catheter 1 is same as described for the catheter of the first embodiment and may be omitted therefore.

## Claims

1. Computer comprising
- first connection means adapted to connect said computer to pump driving means (27) adapted to drive a pump (26) for increasing/decreasing the gas volume V inside at least one inflatable and deflatable balloon (6, 8) of an enteral feeding catheter for channelling flowable nutrient into a patient's digestive tract, said catheter (1) being adapted to be advanced through patient's esophagus,
having a first lumen (2) having an opening (3) at a proximal end (P) of said catheter adapted to be connected to a supply for receiving said nutrient from said supply and channelling said nutrient from said proximal end (P) to a distal end (D) of said catheter (1) being provided with at least one outlet (4) for the exit of said nutrient into patient's digestive tract,
and further comprising a second lumen (7) being connected to one of said inflatable and deflatable balloons (6) provided on said catheter (1) near said distal end (D), and being connected at its proximal end to said pump and a gas pressure gauge (24) adapted to determine the pressure p prevailing in said at least one balloon (6, 8),
- second connection means adapted to connect said computer to said pressure gauge (24),
- calculation means for calculating the mathematical derivative dp/dV of pressure p with respect to volume V and
- accessing means to access executable instructions to cause said computer to cause said pump driving means (27) to adjust the gas volume inside said balloon (6, 8) for initial setting such that said mathematical derivative dp/dV of pressure p with respect to volume V is zero or as close to zero as possible.

2. Computer as claimed in claim 1 further comprising third connection means adapted to connect said computer to valve means (22, 25) adapted to connect for initial settings said pressure gauge (24) to ambient air and disconnect it from ambient air, said accessing means access instructions to cause said computer to connect said pressure gauge (24) to ambient air and to adjust said pressure gauge to zero.

3. Computer as claimed in claim 1 or 2 further comprising alarm means alerting of a blocked or occluded catheter lumen, in case that the mathematical derivative dp/dV of pressure p with respect to volume V exceeds a certain upper threshold at a certain pressure.

4. Computer as claimed in any one of the preceding claims further comprising alarm means alerting of a leakage in case that the mathematical derivative dp/dV of pressure p with respect to volume V fails to reach a lower threshold at a certain pressure.

5. Computer program, especially computer program stored on a machine-readable storage medium, for carrying out pressure measurements in an enteral feeding catheter (1) comprising instructions executable by a computer as claimed in any of the preceding claims to cause said computer for initial settings to drive the pump (26) for increasing/ decreasing the gas volume V inside at least one balloon (6, 8) of said enteral feeding catheter (1) and collecting pressure readings of pressure p prevailing in said at least one balloon (6, 8), calculating the mathematical derivative dp/dV of pressure p with respect to volume V and adjusting the gas volume inside said balloon (6, 8) such that said mathematical derivative dp/dV of pressure p with respect to volume V is zero or as close to zero as possible.

6. Computer program as claimed in claim 5 further comprising the steps of connecting said pressure gauge (24) to ambient air, adjusting said pressure gauge to zero and disconnecting said pressure gauge from ambient air.

7. Computer program as claimed in claim 5 or claim 6 further comprising the step of activating alarm means alerting of a blocked or occluded catheter lumen, in case that the mathematical derivative dp/dV of pressure p with respect to volume V exceeds a certain upper threshold at a certain pressure.

8. Computer program as claimed in any one of claims 5 to claim 7 further comprising the step of activating alarm means alerting of a leakage in a catheter lumen (6, 8), in case that the mathematical derivative dp/dV of pressure p with respect to volume V fails to reach a lower threshold at a certain pressure.

9. Computer program as claimed in anyone of claims 5 to claim 8 further comprising the steps of continuously collecting pressure readings after initialization has been accomplished.

10. Computer program as claimed in claim 8 further comprising the step of activating an alarm when the pressure exceeds a certain threshold.

11. Computerized system comprising
- a computer as claimed in any of claims 1-4,
- pump driving means (27) connected to said computer via said first connecting means,
- a pump driven by said pump driving means,
- a pressure gauge (24) connected to said computer via said first connecting means and adapted to determine the pressure p prevailing in said at least one balloon (6, 8)
- an enteral feeding catheter for channelling flowable nutrient into a patient's digestive tract, said catheter (1) being adapted to be advanced through patient's esophagus, having a first lumen (2) having an opening (3) at a proximal end (P) of said catheter adapted to be connected to a supply for receiving said nutrient from said supply and channelling said nutrient from said proximal end (P) to a distal end (D) of said catheter (1) being provided with at least one outlet (4) for the exit of said nutrient into patient's digestive tract,
and further comprising a second lumen (7) being connected to one of said inflatable and deflatable balloons (6) provided on said catheter (1) near said distal end (D), and being connected at its proximal end to said pump and said gas pressure gauge (24).

## Patentansprüche

1. Computer, aufweisend
- erste Verbindungsmittel ausgebildet zum Verbinden des Computers mit Pumpensteuerungsmitteln (27), welche zum Steuern einer Pumpe (26) zur Erhöhung/Erniedrigung des Gasvolumens V innerhalb mindestens eines aufblasbaren und entleerbaren Ballons (6, 8) einer enteralen Nahrungssonde zum Leiten fließfähiger Nahrung in den Verdauungstrakt eines Patienten angepasst sind, wobei die Sonde (1) zum Vorschieben durch die Speiseröhre des Patienten ausgebildet ist,
aufweisend ein erstes Lumen (2), welches eine Öffnung (3) am proximalen Ende (P) der Sonde besitzt, dazu ausgebildet, um mit einer Zufuhr zum Aufnehmen der Nahrung aus der Zufuhr verbunden zu werden, und um die Nahrung vom proximalen Ende (P) zu einem distalen Ende (D) der Sonde (1) zu leiten, welches über mindestens einen Auslass (4) zum Austritt der Nahrung in den Verdauungstrakt des Patienten versehen ist,
und weiterhin aufweisend ein zweites Lumen (7), welches verbunden ist mit einem der an der Sonde nahe des distalen Endes (D) vorgesehenen, aufblasbaren und entleerbaren Ballons (6), und welches an seinem proximalen Ende mit der Pumpe und einem Gasdruckmesser (24) verbunden ist, welcher zur Bestimmung des vorherrschenden Drucks p in dem mindestens einen Ballon (6, 8) ausgebildet ist,
- zweite Verbindungsmittel, ausgebildet zum Verbinden des Computers mit dem Druckmesser (24),
- Berechnungsmittel zum Berechnen der mathematischen Ableitung dp/dV des Drucks p in Bezug auf das Volumen V und
- Zugriffsmittel zum Zugreifen auf ausführbare Instruktionen, welche den Computer veranlassen, die Pumpensteuerungsmittel (27) zu veranlassen, das Gasvolumen innerhalb des Ballons (6, 8) zur Anfangseinstellung so einzustellen, dass die mathematische Ableitung dp/dV des Drucks p in Bezug auf das Volumen V gleich null oder so nahe null ist wie möglich.

2. Computer gemäß Anspruch 1, weiter aufweisend dritte Verbindungsmittel, die dazu ausgebildet sind, den Computer mit Ventilmitteln (22, 25) zu verbinden, welche angepasst sind, für Anfangseinstellungen den Druckmesser (24) mit der Umgebungsluft zu verbinden und von der Umgebungsluft zu trennen, wobei die Zugriffsmittel auf Instruktionen zugreifen, welche den Computer veranlassen, den Druckmesser (24) mit Raumluft zu verbinden und den Druckmesser auf null einzustellen.

3. Computer gemäß Anspruch 1 oder 2, weiter aufweisend Alarmmittel zum Warnen vor einem blockierten oder verschlossenen Sondenlumen in dem Fall, dass die mathematische Ableitung dp/dV des Drucks p in Bezug auf das Volumen V eine bestimmte obere Schwelle bei einem bestimmten Druck überschreitet.

4. Computer gemäß einem der vorangehenden Ansprüche, weiter aufweisend Alarmmittel, zum Warnen vor einem Leck in dem Fall, dass die mathematische Ableitung dp/dV des Drucks p in Bezug auf das Volumen V eine untere Schwelle bei einem bestimmten Druck nicht erreicht.

5. Computerprogramm, insbesondere Computerprogramm gespeichert auf einem maschinenlesbaren Speichermedium, zur Ausführung von Druckmessungen in einer enteralen Nahrungssonde (1), welche Instruktionen aufweist, ausführbar durch einen Computer gemäß einem der vorangehenden Ansprüche, um den Computer zu veranlassen, für Anfangseinstellungen die Pumpe (26) zum Erhöhen/Erniedrigen des Gasvolumens V innerhalb mindestens eines Ballons (6, 8) der besagten enteralen Nahrungssonde (1) zu steuern, und Druckmessungen des vorherrschenden Drucks p in besagtem, mindestens einem Ballon (6, 8) aufzunehmen, die mathematische Ableitung dp/dV des Drucks p in Bezug auf das Volumen V zu berechnen und das Gasvolumen innerhalb des Ballons (6, 8) so einzustellen, dass die mathematische Ableitung dp/dV des Drucks p in Bezug auf das Volumen V gleich null ist oder so nahe null ist wie möglich.

6. Computerprogramm gemäß Anspruch 5, weiter aufweisend die Schritte des Verbindens des Druckmessers (24) mit Umgebungsluft, des Einstellens des Druckmessers auf null und des Abtrennens des Druckmessers von der Raumluft.

7. Computerprogramm gemäß Anspruch 5 oder Anspruch 6, weiter aufweisend den Schritt des Aktivierens von Alarmmitteln zum Warnen vor einem blockierten oder verschlossenen Sondenlumen, in dem Fall, dass die mathematische Ableitung dp/dV des Drucks p in Bezug auf das Volumen V eine bestimmte obere Schwelle bei einem bestimmten Druck überschreitet.

8. Computerprogramm gemäß einem der Ansprüche 5 bis 7, weiter aufweisend den Schritt des Aktivierens von Alarmmitteln zum Warnen vor einem Leck in einem Sondenlumen (6, 8), in dem Fall, dass die mathematische Ableitung dp/dV des Drucks p in Bezug auf das Volumen V eine untere Schwelle bei einem bestimmten Druck nicht erreicht.

9. Computerprogramm gemäß einem der Ansprüche 5 bis 8, weiter aufweisend die Schritte des fortlaufenden Aufnehmens von Druckmessungen nach vollzogener Initialisierung.

10. Computerprogramm gemäß Anspruch 8, weiter aufweisend den Schritt des Aktivierens eines Alarms, wenn der Druck eine bestimmte Schwelle überschreitet.

11. Computergestütztes System, umfassend
- einen Computer gemäß einem der Ansprüche 1 bis 4,
- Pumpensteuerungsmittel (27) verbunden mit dem Computer über die ersten Verbindungsmittel,
- eine von den Pumpensteuerungsmitteln gesteuerte Pumpe,
- einen Druckmesser (24) verbunden mit dem Computer über die ersten Verbindungsmittel und ausgebildet zum Bestimmen des in dem mindestens einem Ballon (6, 8) vorherrschenden Drucks p,
- eine enterale Nahrungssonde zum Leiten fließfähiger Nahrung in das Verdauungssystem eines Patienten, wobei die Sonde (1) ausgebildet ist zum Vorschieben durch die Speiseröhre des Patienten,
aufweisend ein erstes Lumen (2), welches eine Öffnung (3) am proximalen Ende (P) der Sonde besitzt und dazu ausgebildet ist, mit einer Zufuhr zum Aufnehmen der Nahrung aus der Zufuhr verbunden zu werden und um die Nahrung vom proximalen Ende (P) zu einem distalen Ende (D) der Sonde (1) zu leiten, welches über mindestens einen Auslass (4) zum Austritt der Nahrung in den Verdauungstrakt des Patienten versehen ist,
und weiterhin aufweisend ein zweites Lumen (7), welches verbunden ist mit einem der an der Sonde nahe dem distalen Ende (D) vorgesehenen, aufblasbaren und entleerbaren Ballons (6), und welches an seinem proximalen Ende mit der Pumpe und dem Gasdruckmesser (24) verbunden ist.

## Revendications

1. Ordinateur comprenant :
- des premiers moyens de connexion adaptés pour connecter ledit ordinateur à des systèmes de commande de pompe (27) adaptés pour commander une pompe (26) afin d'augmenter/diminuer le volume de gaz V à l'intérieur d'au moins un ballonnet gonflable et dégonflable (6, 8) d'un cathéter d'alimentation entérale permettant de canaliser des nutriments fluides dans les voies digestives d'un patient, ledit cathéter (1) étant adapté pour être avancé à travers l'oesophage du patient,
doté d'une première lumière (2) ayant une ouverture (3) à une extrémité proximale (P) dudit cathéter, adaptée pour être connectée à une source d'alimentation afin de recevoir lesdits nutriments de ladite source d'alimentation et de canaliser lesdits nutriments depuis ladite extrémité proximale (P) jusqu'à une extrémité distale (D) dudit cathéter (1), dotée d'au moins une sortie (4) pour la sortie desdits nutriments dans les voies digestives du patient,
et comprenant en outre une seconde lumière (7) connectée à un desdits ballonnets gonflables et dégonflables (6) agencés sur ledit cathéter (1) près de ladite extrémité distale (D), et connectée à son extrémité proximale à ladite pompe et à un manomètre de gaz (24), adapté pour déterminer la pression p régnant dans ledit au moins un ballonnet (6, 8),
- des seconds moyens de connexion adaptés pour connecter ledit ordinateur audit manomètre (24),
- des moyens de calcul pour calculer la dérivée mathématique dp/dV de la pression p relativement au volume V et
- des moyens d'accès pour accéder à des instructions exécutables, afin de faire en sorte que ledit ordinateur commande l'ajustement, par lesdits moyens de commande de pompe (27), du volume de gaz à l'intérieur dudit ballonnet (6, 8) pour un paramétrage initial, de sorte que ladite dérivée mathématique dp/dV de la pression p relativement au volume V soit égale à zéro ou aussi proche que possible de zéro.

2. Ordinateur selon la revendication 1, comprenant en outre des troisièmes moyens de connexion adaptés pour connecter ledit ordinateur aux systèmes de soupape (22, 25), adaptés pour connecter, pour des paramétrages initiaux, ledit manomètre (24) à l'air ambiant et le déconnecter de l'air ambiant, lesdits moyens d'accès accédant à des instructions pour que l'ordinateur commande la connexion dudit manomètre (24) à l'air ambiant et ajuste ledit manomètre sur zéro.

3. Ordinateur selon la revendication 1 ou 2, comprenant en outre des systèmes d'alarme alertant d'une lumière de cathéter bloquée ou obstruée, si la dérivée mathématique dp/dV de la pression p relativement au volume V dépasse un seuil supérieur déterminé à une pression déterminée.

4. Ordinateur selon l'une quelconque des revendications précédentes, comprenant en outre des systèmes d'alarme alertant d'une fuite si la dérivée mathématique dp/dV de la pression p relativement au volume V n'atteint pas un seuil inférieur à une pression déterminée.

5. Programme informatique, en particulier un programme informatique stocké sur un support de stockage lisible par machine, pour réaliser des mesures de pression dans un cathéter d'alimentation entérale (1), comprenant des instructions exécutables par un ordinateur, selon l'une quelconque des revendications précédentes, faisant en sorte que ledit ordinateur commande la pompe (26), pour des paramétrages initiaux, afin d'augmenter/diminuer le volume de gaz V à l'intérieur d'au moins un ballonnet (6, 8) dudit cathéter d'alimentation entérale (1) et qu'il récupère les relevés de pression de la pression p régnant dans ledit au moins un ballonnet (6,8), calcule la dérivée mathématique dp/dV de la pression p relativement au volume V et ajuste le volume de gaz à l'intérieur dudit ballonnet (6, 8), de sorte que ladite dérivée mathématique dp/dV de la pression p relativement au volume V soit égale à zéro ou aussi proche que possible de zéro.

6. Programme informatique selon la revendication 5, comprenant en outre les étapes consistant à connecter ledit manomètre (24) à l'air ambiant, ajuster ledit manomètre sur zéro et déconnecter ledit manomètre de l'air ambiant.

7. Programme informatique selon la revendication 5 ou 6, comprenant en outre l'étape consistant à activer des systèmes d'alarme alertant d'une lumière de cathéter bloquée ou obstruée, si la dérivée mathématique dp/dV de la pression p relativement au volume V dépasse un seuil supérieur déterminé, à une pression déterminée.

8. Programme informatique selon l'une quelconque des revendications 5 à 7, comprenant en outre l'étape consistant à activer des systèmes d'alarme alertant d'une fuite dans une lumière de cathéter (6, 8) si la dérivée mathématique dp/dV de la pression p relativement au volume V n'atteint pas un seuil inférieur à une pression déterminée.

9. Programme informatique selon l'une quelconque des revendications 5 à 8, comprenant en outre les étapes consistant à collecter de manière continue les relevés de pression après que l'initialisation a été réalisée.

10. Programme informatique selon la revendication 8, comprenant en outre l'étape consistant à activer une alarme quand la pression dépasse un seuil déterminé.

11. Système informatisé comprenant :
- un ordinateur selon l'une quelconque des revendications 1 à 4,
- des moyens de commande de pompe (27) connectés audit ordinateur par le biais desdits premiers moyens de connexion,
- une pompe commandée par lesdits moyens de commande de pompe,
- un manomètre (24) connecté audit ordinateur, par le biais desdits premiers moyens de connexion et adapté pour déterminer la pression p régnant dans ledit au moins un ballonnet (6, 8),
- un cathéter d'alimentation entérale pour canaliser des nutriments fluides dans les voies digestives d'un patient, ledit cathéter (1) étant adapté pour être avancé à travers l'oesophage du patient, ayant une première lumière (2) dotée d'une ouverture (3) à une extrémité proximale (P) dudit cathéter, adaptée pour être connectée à une source d'alimentation, afin de recevoir lesdits nutriments de ladite source d'alimentation et canaliser lesdits nutriments depuis l'extrémité proximale (P) jusqu'à une extrémité distale (D) dudit cathéter (1), doté d'au moins une sortie (4) pour la sortie desdits nutriments dans les voies digestives du patient,
et comprenant en outre une seconde lumière (7) connectée à l'un desdits ballonnets gonflables et dégonflables (6), agencés sur ledit cathéter (1) près de ladite extrémité distale (D), et connectée à son extrémité proximale à ladite pompe et audit manomètre de gaz (24).
